# EUROPEAN PATENT APPLICATION

(11) **EP 3 361 467 A1**
(43) Date of publication of application: **15.08.2018**
(21) Application number: 17182457.6
(22) Date of filing: 20.07.2017
(51) Int. Cl.: G09B 7/04, A61B 5/16

(54) **INTERACTIVE AND ADAPTIVE TRAINING AND LEARNING MANAGEMENT SYSTEM USING FACE TRACKING AND EMOTION DETECTION WITH ASSOCIATED METHODS**

(30) Priority: 14.02.2017 US 201762458654 P; 15.06.2017 US 201762520542 P
(71) Applicant: Find Solution Artificial Intelligence Limited, Hong Kong (HK)
(72) Inventor: DOLSMA, Johan Matthijs, Hong Kong (HK); LAM, Yuen Lee Viola, Hong Kong (HK)
(74) Representative: de Arpe Fernandez, Manuel

(57) **Abstract**

A system for delivering and managing learning and training programmes comprising optical sensors for capturing subject's facial expression, eye movements, point-of-gaze, and head pose of a student subject during a learning session; a domain knowledge data repository comprising concept data entities, each having knowledge and skill content items, and task data entities, each having lecture content material items; a student module configured to estimate the student subject's affective state and cognitive state using the sensory data collected from the optical sensors; and a trainer module configured to select a task data entity for delivery and presentment to the student subject after each completion of a task data entity based on a probability of the student subject's understanding of the associated concept data entity's knowledge and skill content items; wherein the probability of the student subject's understanding is computed using the student subject's estimated affective state and cognitive state.

## Description

### Cross-references to Related Applications:

This application claims priority to U.S. Patent Application No. 62/458,654 filed February 14, 2017, and U.S. Patent Application No. 62/520,542 filed June 15, 2017; the disclosures of which are incorporated by reference in their entirety.

### Field of the Invention:

The present invention relates generally to methods and systems for providing and delivery of educational programmes and training, including corporate training, academic tutoring, in-class and out-of-class learnings. Particularly, the present invention relates to the customization of tests and assessment of learning progress through the use of emotion detection and analysis.

### Background of the Invention:

In the current education system, especially in South-East Asia, pressure to excel in schools keeps mounting. In a result-oriented based society, the student needs to achieve high grades to have a decent opportunity to enter prestigious academia and obtain advance degrees. The school system continues to rely heavily on class based lecturing combined with paper based examinations. Hence, there is limited support for personalized learning. Furthermore, the knowledge of each student is only evaluated during examinations held a few times per year.

These shortcomings have led to the rise of tutoring centers where there are more personal attentions and dialogues between the teachers/tutors and students. Any deviations from the learning path, or knowledge gaps can then be remedied directly as such. However, in the tutoring industry, good quality teachers/tutors are in scarce supply and teacher/tutor training is often done informally and casually on the job. Pressure on fees has further increased pressure on teachers/tutors to take up more and more related administrative tasks such as course material preparation, class scheduling, and other logistics, reducing effecting teaching time.

The last decades have seen an increased focus on the diagnosis and understanding of psychological disorders such as autism spectrum and attention deficit hyperactivity disorder (ADHD). Parents have high expectations on the ability of educators to spot these disorders in the students, while diagnosing these disorders is difficult and requires professional judgement by the subject matter experts.

To address aforementioned issues, it would be desirable to have an intelligent learning and training system that models the affective and cognitive states of the student, to assist the teacher/trainer in providing personalized instruction, monitor the student's mental health and minimize administrative tasks to let the teacher/trainer focus on teaching/training.

### Summary of the Invention:

The present invention provides a method and a system for delivering and managing interactive and adaptive learning and training programmes using a combination of sensing of the student subject's gestures, emotions, and movements, and quantitative measurements of test results and learning progress. It is also an objective of the present invention to provide such method and system applicable in workplace performance monitoring and appraisal assessment.

In accordance to one aspect of the present invention, the system estimates the affective state and cognitive state of the subject by image and/or video capturing and analyzing the subject's facial expression, eye movements, point-of-gaze, and head pose; and physiologic detection, such as tactile pressure exerted on a tactile sensing device, subject's handwriting, and tone of voice during a sampling time window. The image or video capture can be performed by using built-in or peripheral cameras in desktop computers, laptop computers, tablet computers, and/or smartphones used by the subject, and/or other optical sensing devices. The captured images and/or videos are then analyzed using machine vision techniques. For example, stalled eye movements, out-of-focus point-of-gaze, and a tilted head pose are signals indicating lack of interest and attention toward the subject matters being presented in the test questions; while a strong tactile pressure detected is a signal indicating anxiety, lack of confidence, and/or frustration in the subject matters being presented in a learning or training session.

In accordance to one embodiment, selected performance data and behavioral data from the subject are also collected in determining the subject's understanding of the learning materials. These selected performance data and behavioral data include, but not limited to, correctness of answers, number of successful and unsuccessful attempts, number of toggling between given answer choices, and response speed to test questions of certain types, subject matters, and/or difficulty levels, and working steps toward a solution. For example, the subject's excessive toggling between given choices and slow response speed in answering a test question indicate doubts and hesitations on the answer to the test question. The subject's working steps toward a solution to a test problem are captured for matching with the model solution and in turn provides insight to the subject's understanding of the materials.

The affective state and cognitive state estimation and performance data are primarily used in gauging the subject's understanding of and interests in the materials covered in a learning or training programme. While a single estimation is used in providing a snapshot assessment of the subject's progress in the learning or training programmes and prediction of the subject's test results on the materials, multiple estimations are used in providing an assessment history and trends of the subject's progress in the learning or training programme and traits of the subject. Furthermore, the estimated affective states and cognitive states of the subject are used in the modeling of the learning or training programme in terms of choice of subject matter materials, delivery methods, and administration.

In accordance to another aspect of the present invention, the method and system for delivering and managing interactive and adaptive learning and training programmes logically structure the lecture materials and the delivery mechanism data in a learning and training programme as Domain Knowledge, and its data are stored in a Domain Knowledge repository. A Domain Knowledge repository comprises one or more Concept objects and one or more Task objects. Each Concept object comprises one or more Knowledge and Skill items. The Knowledge and Skill items are ordered by difficulty level, and two or more Knowledge and Skill items can be linked to form a Curriculum. In the case where the present invention is applied in a school, a Curriculum defined by the present invention is the equivalence of the school curriculum and there is one-to-one relationship between a Knowledge and Skill item and a lesson in the school curriculum. The Concept objects can be linked to form a logical tree data structure for used in a Task selection process.

Each Task object has various lecture content materials, and is associated with one or more Concept objects in a Curriculum. In accordance to one embodiment, a Task object can be classified as: Basic Task, Interactive Task, or Task with an Underlying Cognitive or Expert Model. Each Basic Task comprises one or more lecture notes, illustrations, test questions and answers designed to assess whether the subject has read all the materials, and instructional videos with embedded test questions and answers. Each Interactive Task comprises one or more problem-solving exercises each comprises one or more steps designed to guide the subject in deriving the solutions to problems. Each step provides an answer, common misconceptions, and hints. The steps are in the order designed to follow the delivery flow of a lecture. Each Task with an Underlying Cognitive or Expert Model comprises one or more problem-solving exercises and each comprises one or more heuristic rules and/or constraints for simulating problem-solving exercise steps delivered in synchronous with a student subject's learning progress. This allows a tailored scaffolding (e.g. providing guidance and/or hints) for each student subject based on a point in a problem set or space presented in the problem-solving exercise.

In accordance to another aspect of the present invention, the method and system for delivering and managing interactive and adaptive learning and training programmes logically builds on top of the Domain Knowledge two models of operation: Student Model and Training Model. Under the Student Model, the system executes each of one or more of the Task objects associated with a Curriculum in a Domain Knowledge in a learning session for a student subject. During the execution of the Task objects, the system measures the student subject's performance and obtain the student subject's performance metrics in each Task such as: the numbers of successful and unsuccessful attempts to questions in the Task, number of hints requested, and the time spent in completing the Task. The performance metrics obtained, along with the information of the Task object, such as its difficulty level, are fed into a logistic regression mathematical model of each Concept object associated with the Task object. This is also called the knowledge trace of the student subject, which is the calculation of a probability of understanding of the material in the Concept object by the student subject. The advantages of the Student Model include that the execution of the Task objects can adapt to the changing ability of the student subject. For non-limiting example, following the Student Model, the system can estimate the amount of learning achieved by the student, estimate how much learning gain can be expected for a next Task, and provide a prediction of the student subject's performance in an upcoming test. These data are then used in the Training Model and enable hypothesis testing to make further improvement to the system, evaluate teacher/trainer quality and lecture material quality.

Under the Training Model, the system receives the data collected from the execution of the Task objects under the Student Model and the Domain Knowledge for making decisions on the learning or training strategy and providing feedbacks to the student subject or teacher/trainer. Under the Training Model, the system is mainly responsible for executing the followings:
1.) Define the entry point for the first Task. Initially all indicators for Knowledge and Skill items are set to defaults, which are inferred from data in either an application form filled by the student subject or teacher/trainer or an initial assessment of the student subject by the teacher/trainer. Select the sequence of Tasks to execute. To select the next Task, the system's trainer module has to search through a logical tree data structure of Concept objects, locate a Knowledge and Skill with the lowest skill level and then use a question matrix to lookup the corresponding Task items that match the learning traits of the student subject. Once selected, the necessary lecture content material is pulled from the Domain Knowledge, and send to the system's communication module for delivery presentation in the system's communication module user interface.
2.) Provide feedback. While the student subject is working on a Task object being executed, the system's trainer module monitors the time spent on each Task step. When a limit is exceeded, feedback is provided as a function of the current affective state of the student subject. For example, this can be an encouraging, empathetic, or challenging message selected from a generic list, or it is a dedicated hint from the Domain Knowledge.
3.) Drive the system's pedagogical agent. The system's trainer module matches the current affective state of the student subject with the available states in the pedagogical agent. Besides providing the affective state information, text messages can be sent to the system's communication module for rendering the pedagogical agent in a user interface.
4.) Decide when a Concept is mastered. As described earlier, under the Student Model, the system estimates the student subject's probability of understanding of the materials in each Concept. Based on a predetermined threshold (e.g. 95%), the teacher/trainer can decide when a Concept is mastered.
5.) Flag student subject's behavior that is recognized to be related to mental disorders. For example, when the system's execution under the Student Model shows anomalies in the sensor data compared to a known historical context and exhibits significant lower learning progress, the system under the Training Model raises a warning notice to the teacher/trainer. It also provides more detailed information on common markers of disorders such as Attention Deficit Hyperactivity Disorder (ADHD) and Autism Spectrum Disorder (ASD).

### Brief Description of the Drawings:

Embodiments of the invention are described in more detail hereinafter with reference to the drawings, in which:

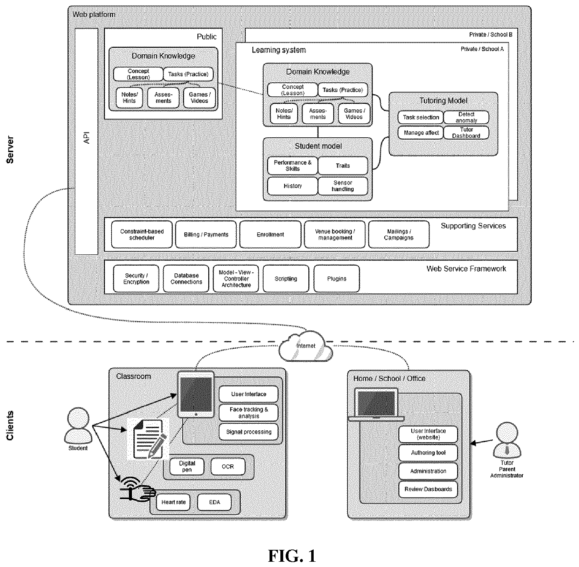

FIG. 1 depicts a schematic diagram of a system for delivering and managing interactive and adaptive learning and training programmes in accordance to one embodiment of the present invention;
FIG. 2 depicts a logical data flow diagram of the system for delivering and managing interactive and adaptive learning and training programmes;
FIG. 3 depicts an activity diagram of a method for delivering and managing interactive and adaptive learning and training programmes in accordance to one embodiment of the present invention;
FIG. 4 depicts a flow diagram of an iterative machine learning workflow used by the system in calculating a probability of understanding of lecture materials by the student subject; and
FIG. 5 illustrates a logical data structure used by the system for delivering and managing interactive and adaptive learning and training programmes in accordance to one embodiment of the present invention.

### Detailed Description:

In the following description, methods and systems for delivering and managing learning and training programmes and the likes are set forth as preferred examples. It will be apparent to those skilled in the art that modifications, including additions and/or substitutions may be made without departing from the scope and spirit of the invention. Specific details may be omitted so as not to obscure the invention; however, the disclosure is written to enable one skilled in the art to practice the teachings herein without undue experimentation.

In accordance to various embodiments of the present invention, the method and system for delivering and managing interactive and adaptive learning and training programmes uses a combination of sensing of the student subject's gestures, emotions, and movements, and quantitative measurements of test results and learning progress.

In accordance to one aspect of the present invention, the system estimates the affective state and cognitive state of the subject by image and/or video capturing and analyzing the subject's facial expression, eye movements, point-of-gaze, and head pose, and haptic feedback, such as tactile pressure exerted on a tactile sensing device during a sampling time window. The image or video capture can be performed by using built-in or peripheral cameras in desktop computers, laptop computers, tablet computers, and/or smartphones used by the subject, and/or other optical sensing devices. The captured images and/or videos are then analyzed using machine vision techniques. For example, stalled eye movements, out-of-focus point-of-gaze, and a tilted head pose are signals indicating lack of interest and attention toward the learning materials being presented in the learning or training session; while a strong tactile pressure detected is a signal indicating anxiety, lack of confidence, and/or frustration in the subject matters being asked in a test question.

In accordance to one embodiment, selected performance data and behavioral data from the subject are also collected in the affective state and cognitive state estimation. These selected performance data and behavioral data include, but not limited to, correctness of answers, number of successful and unsuccessful attempts, toggling between given answer choices, and response speed to test questions of certain types, subject matters, and/or difficulty levels, working steps toward a solution, and the subject's handwriting and tone of voice. For example, the subject's repeated toggling between given choices and slow response speed in answering a test question indicating doubts and hesitations on the answer to the test question. The subject's working steps toward a solution to a test problem are captured for matching with the model solution and in turn provides insight to the subject's understanding of the lecture materials.

In accordance to various embodiments, the system for delivering and managing interactive and adaptive learning and training programmes comprises a sensor handling module implemented by a combination of software and firmware executed in general purposed and specially designed computer processors. The sensor handling module manages the various sensors employed by the system. The sensor handling module is in electrical and/or data communications with various electronic sensing devices including, but not limited to, optical and touch sensing devices; input devices including, but not limited to, keyboard, mouse, pointing device, stylus, and electronic pen; image capturing devices; and cameras.

During the operation of the system, input sensory data are continuously collected at various sampling rates and averages of samples of input sensory data are computed. In order to handle the different sampling rates of different sensing devices, a reference rate is chosen (e.g. 5Hz). A slower sampling rate input sensory data is interpolated with zero order hold and then sampled at the reference rate. A higher sampling rate input sensory data is subsampled at the reference rate. After the sample rate alignment, a trace of the last few seconds is kept in memory after which the average is calculated. Effectively this produces a moving average of an input sensory data and acts as a low-pass filter to remove noise.

### Eye movements, point-of-gaze, and head pose detection

In one embodiment, a low-cost optical sensor built-in in a computing device (e.g. subject facing camera in a tablet computer) is used. At a rate of minimal 5Hz, images are obtained from the sensor. Each image is then processed by face/eye tracking and analysis systems known in the art. The three-dimensional (3D) head orientation is measured in Euler angles (pitch, yaw, and roll). To measure the point-of-gaze, a 3D vector is assumed from the origin of the optical sensor to the center of the pupil of the user, secondly, a 3D vector is determined from the center of the eye-ball to the pupil. These two vectors are then used to calculate the point of gaze. A calibration step helps to compensate for offsets (subject position behind the screen, camera position relative to the screen). Using this data, the planar coordinate of the gaze on the computer screen can be derived.

### Facial expressions and emotions determination

In another embodiment, the images and/or videos captured as mentioned above, are processed to identify key landmarks on the face such as eyes, tip of the nose, corners of the mouth. The regions between these landmarks are then analyzed and classified into facial expressions such as: attention, brow furrow, brow raise, cheek raise, chin raise, dimpler (lip corners tightened and pulled inwards), eye closure, eye widen, inner brow raise, jaw drop, lid tighten, lip corner depression, lip press, lip pucker (pushed forward), lip stretch, lip such, mouth open, nose wrinkle, smile, smirk, upper lip raise. These expressions are then mapped, using a lookup table, onto the following emotions: anger, contempt, disgust, engagement (expressiveness), fear, joy, sadness, surprise and valence (both positive as negative nature of the person's experience). Each emotion is encoded as a percentage and output simultaneously.

### Physiologic measurement

The system may comprise a wearable device to measure physiologic parameters not limiting to: heart rate, electro dermal activity (EDA) and skin temperature. This device is linked wirelessly to the client computing device (e.g. tablet computer or laptop computer). The heart rate is derived from observations of the blood volume pulse. The EDA measures skin conductivity as an indicator for sympathetic nervous system arousal. Based on this, features related to stress, engagement, and excitement can be derived. Another approach is to use vision analysis techniques to directly measure the heart rate based on the captured images. This method is based on small changes in light absorption by the veins in the face, when the amount of blood varies due to the heart rate.

### Handwriting analysis

In another embodiment, test answers may be written on a dedicated note paper using a digital pen and receive commands such as 'step completed'. The written answer is then digitized on the fly and via an intelligent optical character recognition engine, the system can evaluate the content written by the student subject and provide any necessary feedback to guide the student when needed. Studies show that taking longhand notes encourages students to process and reframe information improving the learning results. Alternatively, embodiments may use OCR after the tasks has been completed. The paper is scanned using a copier and the digitized image is fed to OCR software.

### Pedagogical agent-subject interaction

As a non-limiting example, a pedagogical agent may be non-human animated character with human traits implemented by a combination of software and/or firmware running in one or more general purposed computer processors and/or specially configured computer processors. It can display the basic emotions by selecting from a set of animations (e.g. animated GIFs), or by using scripted geometric transformation on a static image displayed to the subject in a user interface. Another method is to use SVG based animations. The animation can be annotated with text messages (e.g. displayed in a balloon next to the animation). The text messages are generated by and received from the trainer module of the system. The subject's responses to the pedagogical agent are received by the system for estimating the subject's affective state.

The affective state and cognitive state estimation is primarily used in gauging the subject's understanding of and interests in the materials covered in a learning or training programme. While a single estimation is used in providing a snapshot assessment of the subject's progress in the learning or training programme and prediction of the subject's test results on the materials, multiple estimations are used in providing an assessment history and trends of the subject's progress in the learning or training programme and traits of the subject. Furthermore, the estimated affective states and cognitive states of the subject are used in the modeling of the learning or training programme in terms of choice of subject matter materials, delivery methods, and administration.

### Domain Knowledge

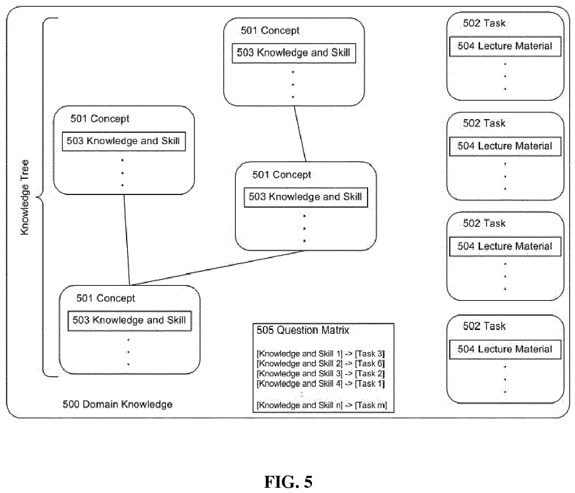

Referring to FIG. 5. In accordance to one aspect of the present invention, the method and system for delivering and managing interactive and adaptive learning and training programmes logically structure the lecture materials, and the delivery mechanism in a learning and training programme as Domain Knowledge **500.** A Domain Knowledge **500** comprises one or more Concept objects **501** and one or more Task objects **502.** Each Concept object **501** comprises one or more Knowledge and Skill items **503.** The Knowledge and Skill items **503** are ordered by difficulty levels, and two or more Concept objects **501** can be grouped to form a Curriculum. In the case where the present invention is applied in a school, a Curriculum defined by the present invention is the equivalence of the school curriculum and there is one-to-one relationship between a Knowledge and Skill item and a lesson in the school curriculum. The Concept objects can be linked to form a logical tree data structure (Knowledge Tree) such that Concept objects having Knowledge and Skill items that are fundamental and/or basic in a topic are represented by nodes closer to the root of the logical tree and Concept objects having Knowledge and Skill items that are more advance and branches of some common fundamental and/or basic Knowledge and Skill items are represented by nodes higher up in different branches of the logical tree.

Each Task object **502** has various lecture content material **504,** and is associated with one or more Concept objects **501** in a Curriculum. The associations are recorded and can be looked up in a lookup matrix **506.** In accordance to one embodiment, a Task object **502** can be classified as: Basic Task, Interactive Task, or Task with an Underlying Cognitive or Expert Model. Each Basic Task comprises one or more lecture notes, illustrations (e.g. video clips and other multi-media content), test questions and answers designed to assess whether the subject has read all the learning materials, and instructional videos with embedded test questions and answers. Each Interactive Task comprises one or more problem-solving exercises each comprises one or more steps designed to guide the subject in deriving the solutions to problems. Each step provides an answer, common misconceptions, and hints. The steps are in the order designed to follow the delivery flow of a lecture. Each Task with an Underlying Cognitive or Expert Model comprises one or more problem-solving exercises and each comprises one or more heuristic rules and/or constraints for simulating problem-solving exercise steps delivered in synchronous with a student subject's learning progress. This allows a tailored scaffolding (e.g. providing guidance and/or hints) for each student subject based on a point in a problem set or space presented in the problem-solving exercise.

In accordance to various embodiments, a Task object gathers a set of lecture materials (e.g. lecture notes, illustrations, test questions and answers, problem sets, and problem-solving exercises) relevant in the achievement of a learning goal. In addition to the aforementioned classification, a Task can be one of the following types:
1.) Reading Task: lecture notes or illustrations to introduce a new topic without grading, required to be completed before proceeding to a Practice Task is allowed;
2.) Practice Task: a set of questions from one topic to practice on questions from a new topic until a threshold is reached (e.g. five consecutive successful attempts without hints, or achieve an understanding level of 60% or more);
3.) Mastery Challenge Task: selected questions from multiple topics to let the student subject achieve mastery (achieve an understanding level of 95% or more) on a topic, and may include pauses to promote retention of knowledge (e.g. review opportunities for the student subjects); or
4.) Group Task: a set of questions, problem sets, and/or problem-solving exercises designed for peer challenges to facilitate more engagement from multiple student subjects, maybe ungraded.

In accordance to one embodiment, the Domain Knowledge, its constituent Task objects and Concept objects, Knowledge and Skill items and Curriculums contained in each Concept object, lecture notes, illustrations, test questions and answers, problem sets, and problem-solving exercises in each Task object are data entities stored a relational database accessible by the system (a Domain Knowledge repository). One or more of Domain Knowledge repositories may reside in third-party systems accessible by the system for delivering and managing interactive and adaptive learning and training programmes.

In accordance to another aspect of the present invention, the method and system for delivering and managing interactive and adaptive learning and training programmes logically builds on top of the Domain Knowledge two models of operation: Student Model and Training Model.

### Student Model

Under the Student Model, the system executes each of one or more of the Task objects associated with a Curriculum in a Domain Knowledge for a student subject. During the execution of the Task objects, the system measures the student subject's performance and obtain the student subject's performance metrics in each Task such as: the numbers of successful and unsuccessful attempts to questions in the Task, number of hints requested, and the time spent in completing the Task. The performance metrics obtained, along with the information of the Task object, such as its difficulty level, are fed into a logistic regression mathematical model of each Concept object associated with the Task object. This is also called the knowledge trace of the student subject, which is the calculation of a probability of understanding of the material in the Concept object by the student subject. In one embodiment, the calculation of a probability of understanding uses a time-based moving average of student subject's answer grades/scores with lesser weight on older attempts, the number of successful attempts, number of failed attempts, success rate (successful attempts over total attempts), time spent, topic difficulty, and question difficulty.

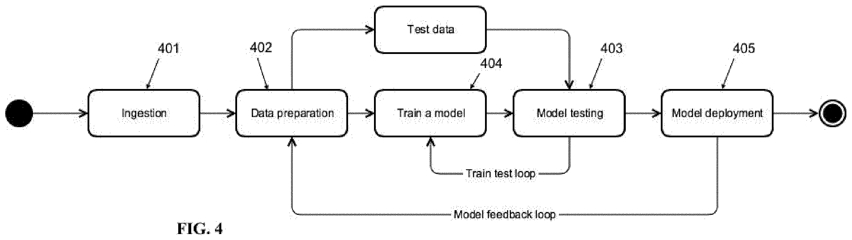

In one embodiment, the system calculates the probability of understanding of the materials in the Concept object by the student subject using an iterative machine learning workflow to fit mathematical models on to the collected data (student subject's performance metrics and information of the Task) including, but not limited to, a time-based moving average of student subject's answer grades/scores with lesser weight on older attempts, the number of successful attempts, number of failed attempts, success rate (successful attempts over total attempts), time spent, topic difficulty, and question difficulty. FIG. 4 depicts a flow diagram of the aforesaid iterative machine learning workflow. In this exemplary embodiment, data is collected (**401**), validated and cleansed (**402**); then the validated and cleansed data is used in attempting to fit a mathematical model (**403**); the mathematical model is trained iteratively (**404**) in a loop until the validated and cleansed data fit the mathematical model; then the mathematical model is deployed (**405**) to obtain the probability of understanding of the materials in the Concept object by the student subject; the fitted mathematical model is also looped back to and used in the step of validating and cleansing of the collected data.

The knowledge trace of the student subject is used by the system in driving Task lecture material items (e.g. questions and problem sets) selection, driving Task object (topic) selection, and driving lecture material ranking. The advantages of the Student Model include that the execution of the Task objects can adapt to the changing ability of the student subject. For non-limiting example, under the Student Model the system can estimate the amount of learning achieved by the student, estimate how much learning gain can be expected for the next Task, and provide a prediction of the student subject's performance in an upcoming test. These data are then used in the Training Model and enable hypothesis testing to make further improvement to the system, evaluate teacher/trainer quality and lecture material quality.

### Training Model

Under the Training Model, the system's trainer module receives the data collected from the execution of the Task objects under the Student Model and the Domain Knowledge for making decisions on the learning or training strategy and providing feedbacks to the student subject or teacher/trainer. The system for delivering and managing interactive and adaptive learning and training programmes comprises a trainer module implemented by a combination of software and firmware executed in general purposed and specially designed computer processors. In one embodiment, the trainer module resides in one or more server computers. The trainer module is primarily responsible for executing the machine instructions corresponding to the carrying-out of the activities under the Training Model. Under the Training Model, the trainer module executes the followings:
1.) Define the entry points for the Tasks execution. Initially all indicators for Concept Knowledge and Skill items are set to defaults, which are inferred from data in either an application form filled by the student subject or teacher/trainer or an initial assessment of the student subject by the teacher/trainer. Select the subsequent Task to execute. To select the next Task, the system's trainer module searches through a logical tree data structure of Concept objects (Knowledge Tree), locate a Concept Knowledge and Skill with the lowest skill level (closest to the root of the Knowledge Tree) and then use a matching matrix to lookup the corresponding Task object for making the selection. Once selected, the Task object data is retrieved from the Domain Knowledge repository, and send to the system's communication module for delivery presentation.
2.) Provide feedback. While the student subject is working on a Task object being executed, the system's trainer module monitors the time spent on a Task step. When a time limit is exceeded, feedback is provided as a function of the current affective state of the student subject. For example, this can be an encouraging, empathetic, or challenging message selected from a generic list, or it is a dedicated hint from the Domain Knowledge.
3.) Drive the system's pedagogical agent. The system's trainer module matches the current affective state of the student subject with the available states in the pedagogical agent. Besides providing the affective state information, text messages can be sent to the system's communication module for rendering along with the pedagogical agent's action in a user interface displayed to the student subject.
4.) Decide when a Concept is mastered. As described earlier, under the Student Model, the system estimates the student subject's probability of understanding of the materials in each Concept. Based on a predetermined threshold (e.g. 95%), the teacher/trainer can decide when a Concept is mastered.
5.) Flag student subject's behavior that is recognized to be related to mental disorders. For example, when the system's execution under the Student Model shows anomalies in the sensory data compared to a known historical context and exhibits significant lower learning progress, the system under the Training Model raises a warning notice to the teacher/trainer. It also provides more detailed information on common markers of disorders such as Attention Deficit Hyperactivity Disorder (ADHD) and Autism Spectrum Disorder (ASD).

In accordance to various embodiments, the system for delivering and managing interactive and adaptive learning and training programmes further comprises a communication module implemented by a combination of software and firmware executed in general purposed and specially designed computer processors. In one embodiment, one part of the communication module resides and is executed in one or more server computers, and other part of the communication module resides and is executed in one or more client computers including, but not limited to, desktop computers, laptop computers, tablet computers, smartphones, and other mobile computing devices, among which some are dedicated for use by the student subjects and others by teachers/trainers.

The communication module comprises one or more user interfaces designed to present relevant data from the Domain Knowledge and materials generated by the system operating under the Student Model and Training Model to the student subjects and the teachers/trainers. The user interfaces are further designed to facilitate user interactions in capturing user input (textual, gesture, image, and video inputs) and displaying feedback including textual hints and the simulated pedagogical agent's actions. Another important feature of the communication module is to provide an on-screen (the screen of the computing device used by a student subject) planar coordinates and size of a visual cue or focal point for the current Task object being executed. For a non-limiting example, when a lecture note from a Task object is being displayed on screen, the communication module provides the planar coordinates and size of the lecture note display area and this information is used to match with the collected data from a point-of-gaze tracking sensor in order to determine whether the student subject is actually engaged in the Task (looking at the lecture note).

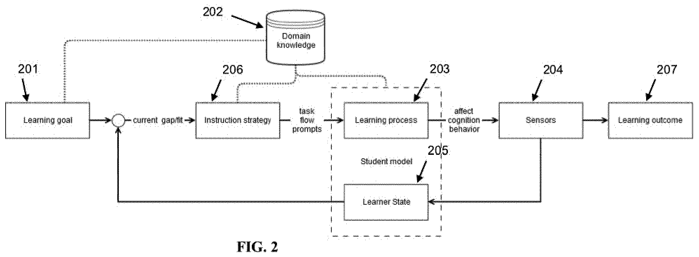

FIG. 2 depicts a logical data flow diagram of the system for delivering and managing interactive and adaptive learning and training programmes in accordance to various embodiments of the present invention. The logical data flow diagram illustrates how the major components of the system work together in a feedback loop in the execution during the Student Model and Training Model. In an exemplary embodiment in reference to FIG. 2, during enrollment, a suitable course is selected by the student (or parents) in a learning or training programme. This course corresponds directly to a Curriculum object, which is a set of linked Concept objects in the Domain Knowledge **202,** and constitutes the learning goal **201** for this student subject. Upon the student subject logging into the system via a user interface rendered by the system's communication module, under the Training Model, the system's trainer module selects and retrieves from the Domain Knowledge **202** a suitable Concept object and the associated first Task object. Entering the Student Model, the Task object data is retrieved from the Domain Knowledge repository, the system renders the Task object data (e.g. lecture notes, test questions, and problem set) on the user interface for the student subject, and the student subject starts working on the task. Meanwhile, the system monitors the learning process **203** by collecting affective state sensory data including, but not limited to, point-of-gaze, emotion, and physiologic data, and cognition state data via Task questions and answers and the student subject's behavioral-analyzing interactions with the user interface (**204**). After analyzing the collected affective state sensory data and cognition state data, the learner state **205** is updated. The updated learner state **205** is compared with the learning goal **201.** The determined knowledge/skill gap or the fit of the instruction strategy **206** is provided to the Training Model again, completing the loop. If the analysis on the collected affective state sensory data and cognition state data shows a probability of understanding that is higher than a threshold, the learning goal is considered achieved **207.**

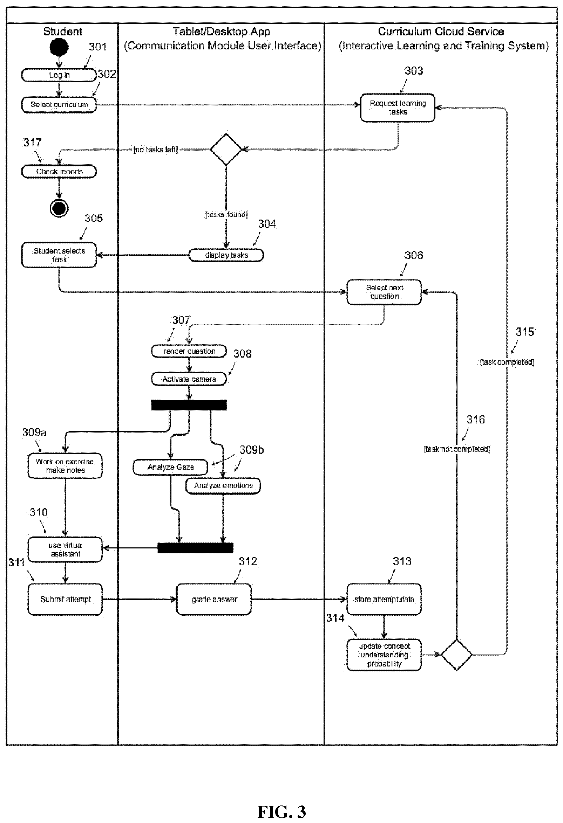

FIG. 3 depicts an activity diagram illustrating in more details the execution process of the system for delivering and managing interactive and adaptive learning and training programmes under the Student Model and Training Model. In an exemplary embodiment referring to FIG. 3, the execution process is as follows:
**301.** A student subject logs into the system via her computing device running a user interface rendered by the system's communication module.
**302.** The student subject select a Curriculum presented to her in the user interface.
**303.** Upon receiving the user login, successful authentication, and receiving the Curriculum selection, the system's trainer module, running in a server computer, selects and requests from the Domain Knowledge repository one or more Task objects associated with the Curriculum selected. When no Task object has yet been defined to associate with any Concept objects in the Curriculum selected, the system evaluates the Knowledge Tree and finds the Concept Knowledge and Skills that have not yet practiced or mastered by the student subject as close to the root (fundamental) of the Knowledge Tree as possible. This process is executed by the system's recommendation engine, which can be implemented by a combination of software and firmware executed in general purposed and specially designed computer processors. The recommendation engine can recommend Practice Tasks, and at lower rate Mastery Challenge Tasks. System-recommended Tasks have a default priority; teachers/trainers-assigned Tasks have a higher priority in the Task selection. In one embodiment, the system further comprises a recommendation engine for recommending the lecture materials (e.g. topic) to be learned next in a Curriculum. Using the estimated affective state and cognitive state data of the student subject, performance data of the student subject, the Knowledge Tree (with all 'edge' topics listed), the teacher/trainer's recommendation information, data from collaborative filters (look at data from peer student subjects), and lecture content data (match student attributes with the learning material's attributes), the recommendation engine recommends the next Task to be executed by the system under the Training Model. For example, the student subject's negative emotion can be eased by recognizing the disliked topics (from the affective state data estimated during the execution of certain Task) and recommending the next Task of a different / favored topic; and recommending the next Task of a disliked topic when student subject's emotion state is detected position. In another example, the recommendation engine can select the next Task of higher difficulty when the estimated affective state data shows that the student subject is unchallenged. This allows the matching of Tasks with the highest learning gains. This allows the clustering of Tasks based on similar performance data and/or affective state and cognitive state estimation. This also allows the matching of student peers with common interests.
**304.** If the requested Task objects are found, their data are retrieved and are sent to the student subject's computing device for presentation in the system's communication module user interface.
**305.** The student subject selects a Task object to begin the learning session.
**306.** The system's trainer module retrieves from the Domain Knowledge repository the next item in the selected Task object for rendering in the system's communication module user interface.
**307.** Entering the Student Model, the system's communication module user interface renders the item in the selected Task object.
**308.** A camera for capturing the student subject's face is activated.
**309.** During the student subject's engagement in learning materials in the item in the selected Task object (**309a**), the student subject's point-of-gaze and facial expressions are analyzed (**309b**).
**310.** Depending on the estimated affective state and cognitive state of the student subject based on sensory data collected and information in the student subject's profile (overlay, includes all past performance data and learning progress data), virtual assistant may be presented in the form of guidance and/or textual hint displayed in the system's communication module user interface.
**311.** The student subject submit an answer attempt.
**312.** The answer attempt is graded and the grade is displayed to the student subject in the system's communication module user interface.
**313.** The answer attempt and grade is also stored by the system for further analysis.
**314.** The answer attempt and grade is used in calculating the probability of the student subject's understanding of the Concept associated with the selected Task object.
**315.** If the selected Task is completed, the system's trainer module selects and requests the next Task based on the calculated probability of the student subject's understanding of the associated Concept and repeat the steps from step **303.**
**316.** If the selected Task is not yet completed, the system's trainer module retrieves the next item in the selected Task and repeat the steps from step **306.**
**317.** After all Tasks are completed, the system generates the result report for student subject.

In accordance to another aspect of the present invention, the system for delivering and managing interactive and adaptive learning and training programmes further comprises an administration module that takes information from the teachers/trainers, student subjects, and Domain Knowledge in offering assistance with the operation of face-to-face learning process across multiple physical education/training centers as well as online, remote learning. In an exemplary embodiment, the administration module comprises a constraint based scheduling algorithm that determines the optimal scheduling of lessons while observing constraints such a teacher/trainer certification, travelling distance for student and trainer, first-come-first-served, composition of the teaching/training group based on learning progress and training strategy. For example, when the teacher/trainer wants to promote peer teaching/training, the scheduling algorithm can select student subjects with complementary skill sets so that they can help each other.

An in-class learning session may comprise a typical flow such as: student subjects check in, perform a small quiz to evaluate the cognitive state of the student subjects, and the results are presented on the teacher/trainer's user interface dashboard directly after completion. The session then continues with class wide explanation of a new concept by the teacher/trainer, here the teacher/trainer receives assistance from the system's pedagogical agent with pedagogical goals and hints. After the explanation, the student subjects may engage with exercises/tasks in which the system provides as much scaffolding as needed. Based on the learning progress and affective states of the student subjects, the system's trainer module decides how to continue the learning session with a few options: e.g. provide educational games to deal with negative emotions, and allow two or more student subjects engage in a small competition for a small prize, digital badge, and the like. The learning session is concluded by checking out. The attendance data is collected for billing purposes and secondly for safety purposes as the parents can verify (or receive a notification from the system) of arrival and departure times of their children.

Although the embodiments of the present invention described above are primarily applied in academic settings, the present invention can be adapted without undue experimentation to corporate training, surveying, and job performance assessment. In accordance to one embodiment of the present invention, the method and system for delivering and managing interactive and adaptive training programmes logically structure training materials and the delivery mechanism data in a training programme as a Domain Knowledge, with its constituent Concept objects and Task objects having Knowledge and Skill items, and training materials respectively that are relevant to the concerned industry or trade. The system's operations under the Student Model and the Training Model are then substantially similar to those in academic settings. In the application of surveying, the system's estimation of the subjects' affective states and cognitive states can be used in driving the selection and presentment of survey questions. This in turn enables more accurate and speedy survey results procurements from the subjects. In the application of job performance assessment, the system's estimation of the employee subjects' affective states and cognitive states on duty continuously allows an employer to gauge the skill levels, engagement levels, and interests of the employees and in turn provides assistance in work and role assignments.

The present invention can also be applied in medical assessment for cognitive disorders, such as Alzheimers' dementia and autism ADHD. In a cognitive test (e.g. administered using a tablet computer to a patient subject), the system estimates the patient subject's affective state and cognitive state using the collected (e.g. from the tablet computer's built-in camera) and analyzed sensory data on patient subject's facial expression, eye movements, point-of-gaze, head pose, voice, speech clarity, reaction time, and/or touch responses. The patient subject's affective state and cognitive state estimation, along with the patient subject's cognitive test performance data are used to drive the course of the cognitive test, influence the patient subject's emotions, and provide a real-time diagnosis that is less prone to human error. The patient subject's affective state and cognitive state estimation can also be matched and used alongside with MRI data on the patient subject's brain activity in further study.

The electronic embodiments disclosed herein may be implemented using general purpose or specialized computing devices, computer processors, or electronic circuitries including but not limited to application specific integrated circuits (ASIC), field programmable gate arrays (FPGA), and other programmable logic devices configured or programmed according to the teachings of the present disclosure. Computer instructions or software codes running in the general purpose or specialized computing devices, computer processors, or programmable logic devices can readily be prepared by practitioners skilled in the software or electronic art based on the teachings of the present disclosure.

All or portions of the electronic embodiments may be executed in one or more general purpose or computing devices including server computers, personal computers, laptop computers, mobile computing devices such as smartphones and tablet computers.

The electronic embodiments include computer storage media having computer instructions or software codes stored therein which can be used to program computers or microprocessors to perform any of the processes of the present invention. The storage media can include, but are not limited to, floppy disks, optical discs, Blu-ray Disc, DVD, CD-ROMs, and magnetooptical disks, ROMs, RAMs, flash memory devices, or any type of media or devices suitable for storing instructions, codes, and/or data.

Various embodiments of the present invention also may be implemented in distributed computing environments and/or Cloud computing environments, wherein the whole or portions of machine instructions are executed in distributed fashion by one or more processing devices interconnected by a communication network, such as an intranet, Wide Area Network (WAN), Local Area Network (LAN), the Internet, and other forms of data transmission medium.

The foregoing description of the present invention has been provided for the purposes of illustration and description. It is not intended to be exhaustive or to limit the invention to the precise forms disclosed. Many modifications and variations will be apparent to the practitioner skilled in the art.

The embodiments were chosen and described in order to best explain the principles of the invention and its practical application, thereby enabling others skilled in the art to understand the invention for various embodiments and with various modifications that are suited to the particular use contemplated.

## Claims

1. A system for delivering and managing learning and training programmes comprising:
one or more optical sensors configured for capturing and generating sensory data on a student subject during a learning session;
one or more electronic databases including one or more domain knowledge data entities, each domain knowledge data entity comprising one or more concept data entities and one or more task data entities, wherein each concept data entity comprises one or more knowledge and skill content items, wherein each task data entity comprises one or more lecture content material items, wherein each task data entity is associated with at least one concept data entity, and wherein a curriculum is formed by grouping a plurality of the concept data entities;
a student module executed by one or more computer processing devices configured to estimate the student subject's affective state and cognitive state using the sensory data collected from the optical sensors;
a trainer module executed by one or more computer processing devices configured to select a subsequent task data entity and retrieve from the electronic databases the task data entity's lecture content material items for delivery and presentment to the student subject after each completion of a task data entity in the learning session; and
a recommendation engine executed by one or more computer processing devices configured to create a list of task data entities available for selection of the subsequent task data entity, wherein the task data entities available for selection are the task data entities associated with the one or more concept data entities forming the curriculum selected;
wherein the selection of a task data entity from the list of task data entities available for selection is based on a probability of the student subject's understanding of the associated concept data entity's knowledge and skill content items; and
wherein the probability of the student subject's understanding is computed using input data of the estimation of the student subject's affective state and cognitive state.

2. The system of claim 1, further comprising:
one or more physiologic measuring devices configured for capturing one or more of the student subject's tactile pressure exerted on a tactile sensing device, heart rate, electro dermal activity (EDA), skin temperature, and touch response, and generating first additional sensory data during a learning session;
one or more voice recording devices configured for capturing the student subject's voice and speech clarity, and generating second additional sensory data during a learning session; and
one or more handwriting capturing devices configured for capturing the student subject's handwriting, and generating third additional sensory data during a learning session;
wherein the student module is further configured to estimate the student subject's affective state and cognitive state using the sensory data collected from the optical sensors, the first additional sensory data collected from the physiologic measuring devices, the second additional sensory data collected from the voice recording devices, and the third additional sensory data collected from the handwriting capturing devices.

3. The system of claim 1, further comprising:
one or more pedagogical agents configured for capturing the student subject's interaction with the pedagogical agents, and generating additional sensory data during a learning session;
wherein the student module is further configured to estimate the student subject's affective state and cognitive state using the sensory data collected from the optical sensors and the additional sensory data collected from the pedagogical agents.

4. The system of claim 1, wherein each of the lecture content material items is a lecture note, an illustration, a test question, a video with an embedded test question, a problem-solving exercise having multiple steps designed to provide guidance in deriving a solution to a problem, or a problem-solving exercise having one or more heuristic rules or constraints for simulating problem-solving exercise steps delivered in synchronous with the student subject's learning progress.

5. The system of claim 1,
wherein a plurality of the concept data entities are linked to form a logical tree data structure;
wherein concept data entities having knowledge and skill content items that are fundamental in a topic are represented by nodes closer to a root of the logical tree data structure and concept data entities having knowledge and skill content items that are advance and branches of a common fundamental knowledge and skill content item are represented by nodes higher up in different branches of the logical tree data structure;
wherein the recommendation engine is further configured to create a list of task data entities available for selection of the subsequent task data entity, wherein the task data entities available for selection are the task data entities associated with the one or more concept data entities forming the curriculum selected and the one or more concept data entities having knowledge and skill items not yet mastered by the student subject and as close to the roots of the logical tree data structures that the concept data entities belonging to.

6. The system of claim 1,
wherein the probability of the student subject's understanding of the associated concept data entity's knowledge and skill content items is computed using input data of the estimation the student subject's affective state and cognitive state and the student subject's performance data and behavioral data; and
wherein the student subject's performance data and behavioral data comprises one or more of correctness of answers, a time-based moving average of student subject's answer grades, number of successful and unsuccessful attempts, number of toggling between given answer choices, and response speed to test questions, top difficulty levels, test question difficulty levels, working steps toward a solution.

7. The system of claim 1, wherein the sensory data comprises one or more of a student subject's facial expression, eye movements, point-of-gaze, and head pose.

8. The system of claim 1,
wherein the selection of a task data entity from the list of task data entities available for selection is based on a probability of the student subject's understanding of the associated concept data entity's knowledge and skill content items and the student subject's estimated affective state;
wherein when the student subject's estimated affective state indicates a negative emotion, a task data entity that is associated with a concept data entity having knowledge and skill content items that are favored by the student subject is selected over another task data entity that is associated with another concept data entity having knowledge and skill content items that are disliked by the student subject; and
wherein when the student subject's estimated affective state indicates a positive emotion, a task data entity that is associated with a concept data entity having knowledge and skill content items that are disliked by the student subject is selected over another task data entity that is associated with another concept data entity having knowledge and skill content items that are favored by the student subject.

9. A method for delivering and managing learning and training programmes comprising:
capturing and generating sensory data on a student subject using one or more optical sensors during a learning session, wherein the sensory data comprises one or more of a student subject's facial expression, eye movements, point-of-gaze, and head pose;
providing one or more electronic databases including one or more domain knowledge data entities, each domain knowledge data entity comprising one or more concept data entities and one or more task data entities, wherein each concept data entity comprises one or more knowledge and skill content items, wherein each task data entity comprises one or more lecture content material items, wherein each task data entity is associated with at least one concept data entity, and wherein a curriculum is formed by grouping a plurality of the concept data entities;
estimating the student subject's affective state and cognitive state using the sensory data collected from the optical sensors; and
selecting a subsequent task data entity and retrieving from the electronic databases the task data entity's lecture content material items for delivery and presentment to the student subject after each completion of a task data entity in the learning session;
creating a list of task data entities available for selection of the subsequent task data entity, wherein the task data entities available for selection are the task data entities associated with the one or more concept data entities forming the curriculum selected;
wherein the selection of a task data entity from the list of task data entities available for selection is based on a probability of the student subject's understanding of the associated concept data entity's knowledge and skill content items; and
wherein the probability of the student subject's understanding is computed using input data of the estimation of the student subject's affective state and cognitive state.

10. The method of claim 9, further comprising:
capturing and generating first additional sensory data on one or more of the student subject's tactile pressure exerted on a tactile sensing device, heart rate, electro dermal activity (EDA), skin temperature, and touch response during a learning session;
capturing and generating second additional sensory data on the student subject's voice and speech clarity using one or more voice recording devices during a learning session; and
capturing and generating third additional sensory data on the student subject's handwriting using one or more handwriting capturing devices during a learning session;
wherein the estimation of the student subject's affective state and cognitive state uses the sensory data collected from the optical sensors, the first additional sensory data, the second additional sensory data collected from the voice recording devices, and the third additional sensory data collected from the handwriting capturing devices.

11. The method of claim 9, further comprising:
capturing and generating additional sensory data on the student subject's interaction with one or more pedagogical agents during a learning session;
wherein the estimation of the student subject's affective state and cognitive state uses the sensory data collected from the optical sensors and the additional sensory data collected from the pedagogical agents.

12. The method of claim 9, wherein each of the lecture content material items is a lecture note, an illustration, a test question, a video with an embedded test question, a problem-solving exercise having multiple steps designed to provide guidance in deriving a solution to a problem, or a problem-solving exercise having one or more heuristic rules or constraints for simulating problem-solving exercise steps delivered in synchronous with the student subject's learning progress.

13. The method of claim 9,
wherein a plurality of the concept data entities are linked to form a logical tree data structure;
wherein concept data entities having knowledge and skill content items that are fundamental in a topic are represented by nodes closer to a root of the logical tree data structure and concept data entities having knowledge and skill content items that are advance and branches of a common fundamental knowledge and skill content item are represented by nodes higher up in different branches of the logical tree data structure;
wherein the task data entities available for selection are the task data entities associated with the one or more concept data entities forming the curriculum selected and the one or more concept data entities having knowledge and skill items not yet mastered by the student subject and as close to the roots of the logical tree data structures that the concept data entities belonging to.

14. The method of claim 9,
wherein the probability of the student subject's understanding of the associated concept data entity's knowledge and skill content items is computed using input data of the estimation the student subject's affective state and cognitive state and the student subject's performance data and behavioral data; and
wherein the student subject's performance data and behavioral data comprises one or more of correctness of answers, a time-based moving average of student subject's answer grades, number of successful and unsuccessful attempts, number of toggling between given answer choices, and response speed to test questions, top difficulty levels, test question difficulty levels, working steps toward a solution.

15. The method of claim 9,
wherein the selection of a task data entity from the list of task data entities available for selection is based on a probability of the student subject's understanding of the associated concept data entity's knowledge and skill content items and the student subject's estimated affective state;
wherein when the student subject's estimated affective state indicates a negative emotion, a task data entity that is associated with a concept data entity having knowledge and skill content items that are favored by the student subject is selected over another task data entity that is associated with another concept data entity having knowledge and skill content items that are disliked by the student subject; and
wherein when the student subject's estimated affective state indicates a positive emotion, a task data entity that is associated with a concept data entity having knowledge and skill content items that are disliked by the student subject is selected over another task data entity that is associated with another concept data entity having knowledge and skill content items that are favored by the student subject.
